# EUROPÄISCHE PATENTSCHRIFT

(11) **EP 0 487 895 B1**
(45) Veröffentlichungstag und Bekanntmachung des Hinweises auf die Patenterteilung: **11.01.1995**
(21) Anmeldenummer: 91117876.2
(22) Anmeldetag: 19.10.1991
(51) Int. Cl.: A61B 17/58

(54) **Verankerungseinrichtung**
Anchoring device
Dispositif d'ancrage

(30) Priorität: 26.11.1990 CH 3733/90
(43) Veröffentlichungstag der Anmeldung: 03.06.1992
(73) Patentinhaber: Synthes AG, Chur, CH-7002 Chur (CH)
(72) Erfinder: Schläpfer, Johannes Fridolin, CH-8750 Glarus (CH)
(74) Vertreter: Lusuardi, Werther Giovanni, Dr.

(56) Entgegenhaltungen:
- EP-A- 0 242 708
- EP-A- 0 330 881
- EP-A- 0 441 729
- WO-A-89/00028

## Beschreibung

Die Erfindung betrifft eine Verankerungseinrichtung nach dem Oberbegriff des Patentanspruchs 1.

Verankerungseinrichtungen dieser Gattung sind Bestandteile von Systemen, die in der Chirurgie vornehmlich zur Stabilisierung von Wirbelsäulensegmenten verwendet werden. Sie sind entweder als Pedikelschrauben, welche in die Knochensubstanz der Wirbel gesetzt werden, oder als Haken, welche mit ihrer Klinge an den Wirbeln abgestützt werden, ausgebildet und werden mit Längsträgern (Stäbe oder ähnliche Teile) verbunden. (Da Pedikelschrauben und Haken sich bezüglich der in dieser Schrift beschriebenen Eigenschaften nicht unterscheiden, gilt das in dieser Schrift für Pedikelschrauben gesagte auch für Haken und es wird für beides auch die gemeinsame Bezeichnung "Verankerungsteil" verwendet.) Mittels solcher Systeme werden die betreffenden Wirbel entweder in einem festen Abstand zueinander gehalten oder mit entsprechenden, bekannten Mitteln komprimiert, bzw. distrahiert. Je nach Ausbildung solcher Vorrichtungen weist die Verbindung zu den Längsträgern drei oder vier, mit einem einzigen Element blockierbare Freiheitsgrade auf.

Das Ziel bei der Verwendung von Pedikelschrauben liegt in der spannungsfreien Verbindung und in einer aktiven anatomischen Reposition. Je nach Wunsch des Chirurgen kann die Rotation der Pedikelschraube, in der durch die Längsachsen der Pedikelschraube und des Trägers definierten Ebene, vor oder nach der Verschiebung entlang dem Längsträger blockiert werden. Die Pedikelschrauben werden durch die Pedikel in die Wirbelkörper eingeführt.
Das Ziel bei der Verwendung von Haken liegt in einer anatomischen gerechten Positionierung des Hakens. Die Rotation des Hakens ist vor oder nach der Verschiebung entlang dem Längsträger blockierbar. Die Haken werden je nach Bedarf von cranial (Lamina oder Querfortsatz) oder caudal (Pedikel oder Lamina) eingeführt, bzw. eingehängt.

Beim operativen Einbau einer solchen Verankerungseinrichtung ergeben sich folgende Probleme:
- Die Einrichtungen müssen zusammen mit den Stäben in einfacher und wenig zeitaufwendiger Weise einbaubar sein, um dem Patienten vermeidbare Operationszeiten zu ersparen.
- Beim Einbau dürfen die Verankerungsteile (Pedikelschrauben oder Haken) keine unerwünschte Kräfte und Momente auf die tragenden Knochenteile ausüben.

Es liegen eine grosse Anzahl von Konstruktionen solcher Verankerungseinrichtungen vor, welche die obengenannten beiden Eigenschaften anstreben. Dabei sind zwei dieser Lösungen als nächstliegender Stand der Technik zu betrachten:
In dem DE-GM 8 915 443 wird eine Verankerungseinrichtung beschrieben, bei welcher der Aufnahmeteil für die Stäbe, welche mit dem die Befestigung am Knochen bewirkenden Element verbunden ist, eine U-förmige Gestalt besitzt, die auf seiner Innenseite Teile eines Innengewindes trägt.
Der Stab wird in den U-förmigen Aufnahmeteil eingelegt. Dann wird ein Stopfen in das Innengewinde gegen den Stab geschraubt. Der Unterteil des Stopfens und/oder der Stab besitzen eine solche Oberflächenbeschaffenheit, dass eine feste Verbindung des durch das Innengewinde festgehaltenen Stopfens mit dem Stab gewährleistet ist. Diese Verbindung lässt sich zwar während einer Operation sehr schnell herstellen. Der Stab lässt sich aber nur in genau einer Richtung, bezogen auf das Befestigungselement in der Knochensubstanz des Wirbels, festziehen. Sind die beiden Befestigungselemente daher nicht sehr genau ausgerichtet, was schwierig erreichbar ist, so entstehen durch das Festziehen der Stopfen an beiden Stabenden unerwünschte Spannungen.

Eine gegenüber dem DE-GM 8 915 443 verbesserte Einrichtung ist in der EP-A1 0 330 881 dargestellt. Auch hier ist ein U-förmiger Aufnahmeteil vorgesehen, in den während der Operation nach dem Setzen der Pedikelschrauben oder dem Einhängen der Haken der Stab einlegbar ist. Dieser U-förmige Aufnahmeteil ist so gestaltet, dass er in der Mitte des Scheitels eines U eine nach dem Inneren des U gerichtete Spitze aufweist, auf welche der Stab zu liegen kommt. An den oberen Enden des U ist an dem Inneren der Backen je eine Gleitvorrichtung angebracht. In diesen wird ein Teil eingeschoben, der zwei Schrauben aufweist, die in Achsrichtung des Stabes vor und hinter der Spitze liegen und sich gegen die Achse eindrehen lassen. Liegt der Stab auf den zwei sie tragenden Aufnahmeteilen der Pedikelschrauben, so lässt sich durch vorsichtiges wechselweises Eindrehen der vier Schrauben der Stab ohne unerwünschte Spannungen an den Pedikelschrauben befestigen, dies erfordert aber Zeit und eine gewisse Geschicklichkeit beim Festdrehen, wenn unerwünschte Spannungen vermieden werden sollen.

Der Erfindung liegt die Aufgabe zugrunde, eine Verankerungseinrichtung zu schaffen, mit der die beiden Forderungen:
- Herstellung der Verbindung zwischen den Pedikelschrauben (und/oder Haken) und dem Stab in einfacher Weise bei geringem Aufwand an Operationszeit, und
- Vermeiden von unerwünschten Kräften und Momenten bei der Herstellung dieser Verbindung,
auf bessere Weise zwangsläufig durch konstruktive Massnahmen gelöst werden.

Diese Aufgabe wird erfindungsgemäss durch die im Kennzeichenteil des Patentanspruchs 1 aufgeführten Merkmale gelöst. Die weiteren Ansprüche betreffen vorteilhafte Ausführungsformen.

Die Erfindung wird beispielhaft an Hand der Zeichnung erklärt, wobei eine Ausführungsform mit einer Pedikelschraube als Verankerungsteil dargestellt ist. Sämtliche Vorteile der Erfindung lassen sich auch bei einer Ausführungsform mit einem Haken, beispielsweise wie in dem DE-GM 8 915 443 beschrieben, erreichen.

Es zeigen
- Fig. 1: Eine erfindungsgemässe Verankerungseinrichtung, dabei
1a Schnitt in Stabrichtung
1b Schnitt quer zur Stabrichtung
1c Draufsicht
1d Querbolzen, Ansicht in der Stabrichtung
- Fig. 2: Eine Variation der erfindungsgemässen Verankerungseinrichtung, dabei
2a Schnitt in Stabrichtung
2b Schnitt quer zur Stabrichtung
- Fig. 3: Querschnitt der Mutter
In allen Figuren sind für gleiche Teile gleiche Bezugszeichen verwendet.

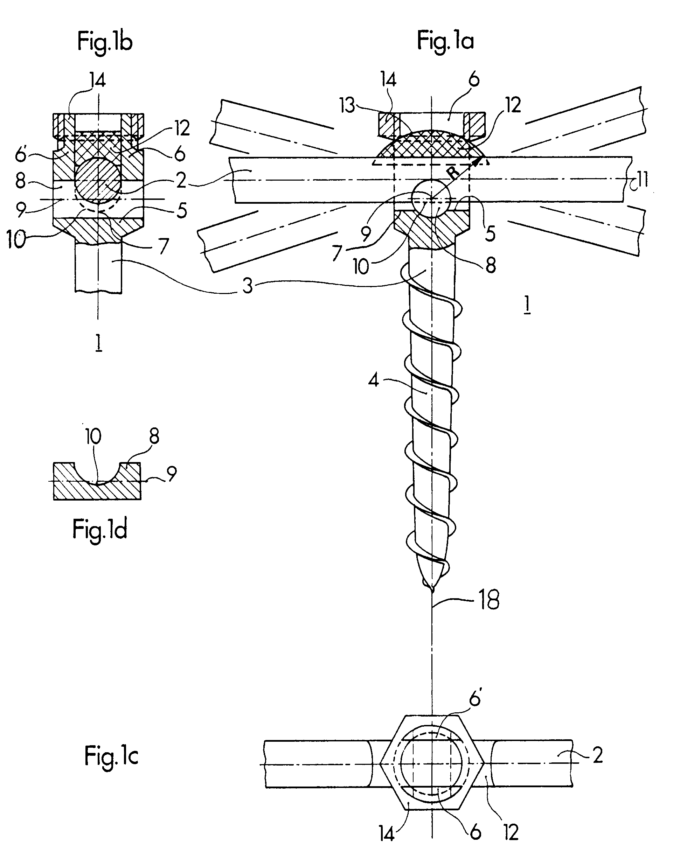

In der Fig. 1 ist ein Teil einer erfindungsgemässen Verankerungseinrichtung 1, im folgenden kurz "Einrichtung" genannt, mit darin befestigtem Stab 2, dargestellt, wobei der Figurenteil 1a einen Schnitt in der Stabrichtung, 1b einen Schnitt quer zur Stabrichtung, 1c eine Draufsicht und 1d einen Querbolzen in einer Ansicht in der Stabrichtung darstellt. Eine zweite Verankerungseinrichtung ist nicht gezeichnet.

Mit der Einrichtung 1 wird ein Stab 2 an mindestens zwei Pedikelschrauben 3 befestigt. Von den Pedikelschrauben 3 ist nur eine gezeichnet. Die Pedikelschrauben 3 setzen sich je aus einem Gewindeschaft 4, der - hier nicht gezeichnet - in die Knochensubstanz beispielsweise eines Wirbels eingeschraubt wird, und einem Aufnahmeteil 5 zusammen, in den der Stab 2 einlegbar und in diesem befestigbar ist. Dazu ist der Aufnahmeteil 5, in Richtung des Stabes 2 gesehen, U-förmig ausgebildet, wobei die oberen Enden des U durch Backen 6,6' gebildet werden. Die Scheitellinie, bzw. Scheitelebene des U ist mit 7 bezeichnet. In den Aufnahmeteil 5 ist senkrecht zur Richtung der Pedikelschraube 3 und zur Scheitellinie 7 des Aufnahmeteils 5 ein Querbolzen 8 seitlich eingesetzt, der senkrecht zu seiner Achse 9 einen Ausschnitt 10 in der Form eines Zylindersegments besitzt, das denselben Radius wie der Stab 2 aufweist. Der Stab 2 passt sich nach seiner Aufnahme in das U des Aufnahmeteils 5 in diesen Ausschnitt 10 ein. Die untere Begrenzung des U liegt tiefer als die untere Begrenzung des Ausschnitts 10. Daher kann der Stab 2 um die Achse 9 des Querbolzens 8 so weit geschwenkt werden wie dies durch diesen Höhenunterschied und die Länge des Aufnahmeteils 5 in der Richtung des Stabes 2 zugelassen wird.
Der Querbolzen 8, bzw. der in einem späteren Abschnitt genannte untere Formteil 16 stellt somit eine Auflage für den Stab 2 dar, die jeweils eine Rotationsachse 9 besitzt, um die der Stab 2 schwenkbar ist.

Beim Einschrauben werden zwei Pedikelschrauben 3 durch Drehen so ausgerichtet, dass der Stab 2 in beide Aufnahmeteile 5 auf die Ausschnitte 10 der Querbolzen 8 aufgelegt werden kann.
Beim Auflegen schwenkt der Stab 2 beide Querbolzen 8, bis er eine Stellung erreicht, in der er in seiner Richtung und quer dazu spannungsfrei an beiden Pedikelschrauben 3 befestigbar ist.

Die Vorrichtung zum Befestigen des Stabs 2 an der Pedikelschraube 3 besteht aus einem oberen Formteil 12 und einer Mutter 14. Der obere Formteil 12 liegt zwischen den Backen 6,6' des Aufnahmeteils 5 auf dem Stab 2 formschlüssig an und besitzt auf der Seite, die dem Stab 2 abgewandt ist, eine Fläche 13 die, wenn richtig positioniert (symmetrisch zur Längsachse 18 der Pedikelschraube 3), zu der Achse 9 des Querbolzens 8 einen Radius R aufweist. Zur leichteren Montage des oberen Formteils 12 kann dieses eine (nicht dargestellte) zentrale Gewindebohrung aufweisen, in welche temporär ein Hilfsinstrument, z.B. in Form eines gewindeten Stabes eingeführt werden kann.

Das Einrichten des oberen Formteils 12 und die Befestigung zwischen dem Stab 2 und der Pedikelschraube 3 erfolgt mit Hilfe einer Mutter 14. Diese wird an Innen-, bzw. Aussengewindeteilen an den Backen 6,6' des Aufnahmeteils 5 geschraubt. Der untere Rand dieser Mutter 14 verläuft nach aussen schräg, dass sich beim Eindrehen der Mutter 14 auf die Gewindeteile der obere Formteil 12 zunächst in die richtige Stellung auf dem Stab 2 verschiebt, in der sich alle Eckpunkte der Fläche 13 im Radius R zu der Achse 9 des Querbolzens 8 befinden und so eine spannungsfreie Befestigung ermöglicht wird. Durch weiteres Anziehen der Mutter 14 wird in dieser Stellung eine klemmende Verbindung zwischen dem oberen Formteil 12, dem Stab 2, dem Querbolzen 8 und dem Aufnahmeteil 5 gebildet und somit ist der Stab 2 spannungsfrei in seiner Richtung und quer dazu an der Pedikelschraube 3 befestigt.

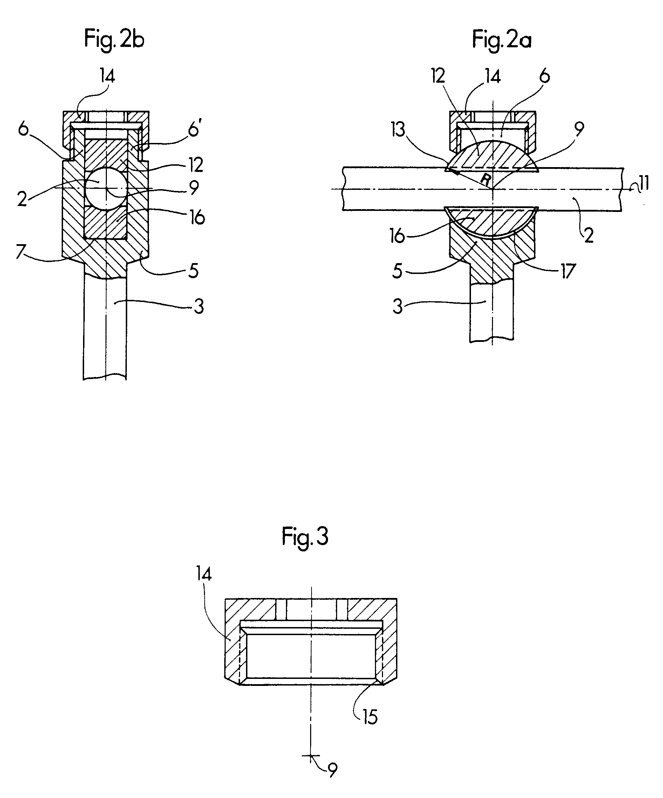

Eine Variante dieser Einrichtung zeigt die Fig. 2, wobei wie oben 2a einen Schnitt in der Stabrichtung und 2b einem Schnitt quer zur Stabrichtung darstellt. Hier liegt die Rotationsachse 9 in Höhe der Stabachse 11. Statt des Querbolzens 8, der (in Fig. 1) in den Aufnahmeteil 5 seitlich eingesetzt wird, ist hier ein unterer Formteil 16 von oben in den Aufnahmeteil 5 eingelegt, der dieselbe Form besitzt wie der oben beschriebene, obere Formteil 12. Er weist eine zylindrische Fläche 17 auf, die ebenfalls den Radius R zur Rotationsachse 9 besitzt.

Der untere Formteil 16 und der obere Formteil 12 können aber beide auch so ausgeführt sein, dass ihre Flächen 13,17 Teile einer Kugel sind, deren Mittelpunkt auf der Stabachse 11 und der Rotationsachse 9 liegt. In diesem Fall muss der Ausschnitt 10 des Aufnahmeteils 5 ebenfalls kugelförmig sein. Die Rotationsachse 9 degeneriert dann zu einem Rotationspunkt. Diese Variante bringt bei der hier beschriebenen Ausführungsform mit einer Pedikelschraube 3 als Verankerungsteil keine besonderen Vorteile, da die Pedikelschraube 3 frei um ihre Längsachse 18 drehbar ist; bei einer Ausführungsform mit einem Haken als Verankerungsteil bietet diese Variante hingegen besondere Vorteile.

Schliesslich können der untere und obere Formteil 16 und 12 an einer ihrer Längsseiten miteinander verbunden sein, so dass ein einteiliges auf der anderen Längsseite offenes, geschlitztes Formteil resultiert, welches gesamthaft auf den Stab 2 aufgeschoben werden kann.

Die in Fig. 3 dargestellte Mutter 14 besitzt eine schräge, rotationssymmetrische Innenfläche 15, die bei richtiger Stellung des oberen Formteils 12 auf gegen die Stabachse 11 möglichst steile Begrenzung der Fläche 13 des oberen Formteils 12 zu liegen kommt. Sie kann daher beim Eindrehen in die auf den Backen 6,6' angebrachten Gewindeteilen das obere Formteil 12 zwangsläufig auf dem Stab 2 in die richtige Stellung verschieben. Der untere Rand der Mutter 14 wird vorteilhafterweise aus hartem gleitfähigem Material hergestellt, das härter ist als die Fläche 13 des oberen Formteils 12. Dreht man nun die Mutter 14 geringfügig weiter, so schneidet oder drückt sich die Mutter 14 in den oberen Formteil 12 und klemmt den Stab 2, den Querbolzen 8, bzw. den unteren Formteil 16 und den Aufnahmeteil 5, so dass eine spannungsfreie Befestigung zwischen dem Stab 2 und der Pedikelschraube 3 entsteht.

Die Entkoppelung der Freiheitsgrade der Verankerungseinrichtung kann auf drei verschiedene Arten erfolgen:
- Verwendung eines Gewindestabes zusammen mit einem glatten Formteil (Blockierung Translation vor Rotation);
- Verwendung eines glatten Stabes mit einem strukturierten, z.B. randrierten Formteil (Blockierung Rotation vor Translation);
- Verwendung eines glatten Stabes mit einem glatten Formteil (Entkoppelung nur mit zusätzlichen Hilfsmitteln möglich).

Die anatomische Reposition umfasst einerseits das Aufrichten der Wirbel (Rotation in der Sagittalebene), anderseits die Kompression, bzw. Distraktion, welche vor oder nach dem Aufrichten der Wirbel und links und rechts separat durchgeführt werden kann. Durch ungleichmässige Distraktion und/oder Kompression links und rechts kann auch eine Rotation der Wirbel in der Frontalebene erreicht werden.
Als Mittel um die Kompression, bzw. Distraktion durchzuführen, seien die folgenden genannt:
a) Verwendung von Spreizer und Kompressor (mit einem sogenannten "Stabblocker";
b) der Stab weist ein rechts- und linksgängiges (separat oder übereinander geschnittenes) Gewinde auf und wird gedreht;
   dabei muss die Fläche 10 des Querbolzens 8, bzw. des unteren Formteils 16 das entsprechende Gewinde aufweisen;
c) der Stab weist ein Gewinde auf, wobei die Kompression, bzw. Distraktion mittels geeigneter Muttern erreicht wird; und
d) Verwendung von zwei Stäben mit einem Rechts-, bzw. Linksgewinde; die verbindende Spannmutter enthält übereinander sowohl ein Links-, als auch ein Rechts-Gewinde.

Die Verankerungseinrichtung nach der Erfindung ist im Laufe einer Operation einfach und in einer wenig zeitaufwendigen Weise einsetzbar, bei ihr können infolge ihrer Konstruktion zwangsläufig keine unerwünschten Spannungen zwischen den Pedikelschrauben 3 und dem Stab 2 auftreten und es ist möglich mit ihr aktiv anatomisch zu reponieren.

Gegenüber den verschiedenen Vorrichtungen gemäss dem Stand der Technik weist die erfindungsgemässe Verankerungseinrichtung 1 einen zusätzlichen Freiheitsgrad auf (bei der Ausführungsform mit kugelförmigen Formteilen sogar zwei zusätzliche Freiheitsgrade). Ein weiterer Vorteil ist, dass sie entweder direkt oder aber über ein Verbindungsglied (beispielsweise gemäss der europ. Patentanmeldung 90116070.5) mit dem Stab 2 verbindbar ist. Damit wird es möglich, die Anzahl der Elemente und die Abstände zwischen Stab 2 und Pedikelschrauben 3 zu minimalisieren.

## Patentansprüche

1. Verankerungseinrichtung (1), oder ein ähnliches Teil wobei an mindestens zwei solcher Verankerungseinrichtungen ein Stab (2) befestigbar ist, bestehend aus einem Verankerungsteil (3) in Form einer Pedikelschraube oder eines Hakens, der einen U-förmigen Aufnahmeteil (5) mit Backen (6,6') und auf diesen Gewindeteile trägt, wobei der Stab (2) in den Aufnahmeteil (5) einlegbar und in diesem befestigbar ist,
dadurch gekennzeichnet,
- dass der Aufnahmeteil (5) eine Auflage (8,16) enthält, die zusammen mit dem Stab (2) um eine senkrecht sowohl zur Längsachse des Verankerungsteils (3) als auch zu einer Scheitellinie (7) des Aufnahmeteils (5) gerichtete Rotationsachse (9) schwenkbar ist,
- dass ein oberer Formteil (12) vorgesehen ist, der formschlüssig auf dem Stab (2) aufsetzbar und in Richtung der Längsachse (11) des Stabes (2) verschiebbar ist, und
- dass der obere Formteil (12) eine Fläche (13) besitzt, die zwangsläufig auf dem Stab (2) in eine Stellung verschoben wird, in der eine in der Längsrichtung und der Querrichtung des Stabes (2) spannungsfreie Verbindung zwischen dem an einem anderen Verankerungsteil befestigten Stab (2) und dem betrachteten Verankerungsteil (3) herstellbar ist, wenn eine Mutter (14) in die Gewindeteile an den Backen (6,6') des Aufnahmeteils (5) festgedreht wird, wozu durch die Mutter (14) beim Eindrehen zunächst der obere Formteil (12) in die genannte Stellung verschoben und beim Festdrehen der Stab (2) am Verankerungsteil (3) fixiert wird.

2. Verankerungseinrichtung (1) nach Anspruch 1, dadurch gekennzeichnet, dass die Auflage als Querbolzen (8) ausgebildet ist.

3. Verankerungseinrichtung (1) nach Anspruch 2, dadurch gekennzeichnet, dass die Rotationsachse (9) die Achse des Querbolzens (8) ist, der in den Aufnahmeteil (5) seitlich eingesetzt ist und einen Ausschnitt (10) in Form eines Zylindersegments besitzt, das denselben Radius aufweist wie der aufzunehmende Stab (2) und dass die Fläche (13) des oberen Formteils (12) zylindrisch ist.

4. Verankerungseinrichtung (1) nach Anspruch 1, dadurch gekennzeichnet, dass die Auflage als unteres Formteil (16) ausgebildet ist.

5. Verankerungseinrichtung (1) nach Anspruch 4, dadurch gekennzeichnet, dass die Rotationsachse (9) in der Höhe der Längsachse (11) des Stabes (2) liegt, wobei der untere Formteil (16) dieselben Abmessungen wie der obere Formteil (12) besitzt, und der untere und der obere Formteil (16,12) je eine entgegengesetzt liegende zylindrische Fläche (17) besitzen, mit denen sie formschlüssig in den Aufnahmeteil (5) einsetzbar sind.

6. Verankerungseinrichtung (1) nach Anspruch 5, dadurch gekennzeichnet, dass der untere und obere Formteil (16;12) gemeinsam durch Drehen hergestellt und dann getrennt oder geschlitzt werden.

7. Verankerungseinrichtung (1) nach nach Anspruch 5 dadurch gekennzeichnet, dass die Flächen (17) des unteren Formteils 16 und/oder des oberen Formteils 12 Kugelabschnitte sind, deren Mittelpunkt Schnittpunkt der Stabachse 11 und der Rotationsachse 9 ist.

8. Verankerungseinrichtung (1) nach einem der Ansprüche 1 bis 7, dadurch gekennzeichnet, dass der untere Rand der Mutter (14) aus weichem, gleitfähigem Material besteht, das weicher ist als die Oberfläche (13) des oberen Formteils (12).

9. Verankerungseinrichtung (1) nach Anspruch 1, dadurch gekennzeichnet, dass die Mutter (14 auf der Seite, mit der sie in die Backen (6;6') eingedreht wird, einen nach aussen schräg verlaufenden Rand besitzt.

10. Verankerungseinrichtung (1) nach einem der Ansprüche 1 bis 9, dadurch gekennzeichnet, dass die Mutter (14) aus einem härteren Material besteht als die Fläche (13) des oberen Formteils (12).

11. Verankerungseinrichtung (1) nach Anspruch 10, dadurch gekennzeichnet, dass die Fläche (13) des oberen Formteils (12) eine Strukturierung, vorzugsweise in Form einer Querrandrierung aufweist.

12. Verankerungseinrichtung (1) nach einem der Ansprüche 3 bis 11, dadurch gekennzeichnet, dass der Ausschnitt (10) des Aufnahmeteils (5) eine glatte Oberfläche aufweist.

13. Verankerungseinrichtung (1) nach einem der Ansprüche 3 bis 12, dadurch gekennzeichnet, dass der Ausschnitt (10) des Aufnahmeteils (5) mit einem relativ zur Längsachse (11) des Stabes (2) rechts- oder linksgängigen Gewinde versehen ist.

14. Vorrichtung zur Stabilisierung von Wirbelsäulensegmenten mit mindestens zwei Verankerungseinrichtungen (1) nach einem der Ansprüche 1 bis 13, dadurch gekennzeichnet, dass die Verankerungseinrichtungen (1) mittels Stäben (2) untereinander verbunden sind.

## Claims

1. An anchoring device (1), whereby a support rod (2) is attachable to at least two of such anchoring devices (1), consisting of an anchoring part (3) in the form of a pedical screw or a pedicle hook having a U-shaped receiving part (5) with jaws (6,6'), said jaws (6,6') being provided with threaded portions, whereby the support rod (2) is insertable into and fixable in the receiving part (5), characterized in that
- the receiving part (5) contains a support element (8,16), which together with the support rod (2) is pivotable around a rotation axis (9) being perpendicular with regard to the longitudinal axis of the anchoring part (3) as well as to an apex line (7) of the receiving part (5),
- an upper positioning element (12) is provided which is form-fittingly positionable on the support rod (2) and slideable in the direction of the longitudinal axis (11) of the support rod (2), and
- the upper positioning element (12) has a surface (13) which is displaced necessarily on the support rod (2) in a position in which a stress-free connection between the support rod (2) attached to another anchoring device (1) and said anchoring part (3) is obtained in the longitudinal direction and in the transverse direction of the support rod (2), when a nut (14) is screwed on the threaded portions of the jaws (6,6') of the receiving part (5), whereby the nut (14) upon screwing on is displacing in a first instance the upper positioning element (12) into said position and upon tightening is fixing the support rod (2) at the anchoring part (3).

2. An anchoring device (1) according to claim 1, characterized in that said support element is designed as a transverse pin (8).

3. An anchoring device (1) according to claim 2, characterized in that said rotation axis (9) is the axis of said transverse pin (8) which is inserted laterally in said receiving part (5) and which has a recess (10) in the shape of a segment of a cylinder having the same radius as the support rod (2) to be received and that said surface (13) of said upper positioning element (12) is cylindrical.

4. An anchoring device (1) according to claim 1, characterized in that said support element is designed as a lower positioning element (16).

5. An anchoring device (1) according to claim 4, characterized in that said rotation axis (9) is positioned at the height of said longitudinal axis (11) of said support rod (2), whereby said lower positioning element (16) has the same dimensions as said upper positioning element (12) and whereby said lower and upper positioning elements (16,12) are provided each with an opposing cylindrical surface (17) by means of which they are form-fittingly insertable into said receiving part (5).

6. An anchoring device (1) according to claim 5, characterized in that said lower and upper positioning elements (16,12) are made uniformly by turning and subsequently divided or slotted.

7. An anchoring device (1) according to claim 5, characterized in that said surfaces (17) of said lower positioning element (16) and/or of said upper positioning element (12) are segments of a sphere the center of which is the intersection of said longitudinal axis (11) with said rotation axis (9).

8. An anchoring device (1) according to one of the claims 1 to 7, characterized in that the lower edge of said nut (14) is made from a soft, slidable material that is softer than said surface (13) of said upper positioning element (12).

9. An anchoring device (1) according to claim 1, characterized in that said nut (14) is provided with a transversally outwardly running edge on the side which is screwed into said jaws (6,6').

10. An anchoring device (1) according to one of the claims 1 to 9, characterized in that said nut (14) consists of harder material than said surface (13) of said upper positioning element (12).

11. An anchoring device (1) according to claim 10, characterized in that said surface (13) of said upper positioning element (12) is provided with a structure, preferably in the form of a transverse knurling.

12. An anchoring device (1) according to one of the claims 3 to 11, characterized in that said recess (10) of said receiving part (5) has a smooth surface.

13. An anchoring device (1) according to one of the claims 3 to 12, characterized in that said recess (10) of said receiving part (5) is provided with right-handed or left-handed thread with respect to said longitudinal axis (11) of said support rod (2).

14. Device for the stabilization of vertebral column segments with at least two anchoring devices (1) according to one of the claims 1 to 13, characterized in that said anchoring devices (1) are interconnected by means of support rods (2).

## Revendications

1. Dispositif d'ancrage (1) ou pièce similaire, une barre (2) pouvant être fixée sur au moins deux tels dispositifs d'ancrage, constitué d'une pièce d'ancrage (3) présentant la forme d'une vis à pédicule ou d'un crochet, qui porte une pièce de réception (5) en forme de U avec mâchoires (6, 6') et des pièces filetées sur ces dernières, la barre (2) pouvant être placée dans la pièce de réception (5) et pouvant y être fixée, caractérisé
- en ce que la pièce de réception (5) contient un appui (8, 16) qui peut être incliné ensemble avec la barre (2) autour d'un axe de rotation (9) orienté perpendiculairement à la fois à l'axe longitudinal de la pièce d'ancrage (3) et à une ligne de sommet (7) de la pièce de réception (5),
- en ce qu'une pièce formée supérieure (12) est prévue, qui peut être posée en correspondance géométrique sur la barre (2) et qui peut être déplacée dans la direction de l'axe longitudinal (11) de la barre (2), et
- en ce que la pièce formée supérieure (12) possède une surface (13) qui est obligatoirement déplacée sur la barre (2) dans une position dans laquelle une liaison sans tension dans la direction longitudinale et dans la direction transversale de la barre (2) peut être réalisée entre la barre (2) fixée sur une autre pièce d'ancrage et la pièce d'ancrage (3) considérée, lorsqu'un écrou (14) est serré dans la partie filetée sur les mâchoires (6, 6') de la pièce de réception (5), la pièce formée supérieure (12) étant tout d'abord déplacée par l'écrou (14) dans ladite position lors de la rotation, et étant immobilisée sur la pièce d'ancrage (3) lors du serrage de la barre (2).

2. Dispositif d'ancrage (1) selon la revendication 1, caractérisé en ce que l'appui présente la forme d'un boulon transversal (8).

3. Dispositif d'ancrage (1) selon la revendication 2, caractérisé en ce que l'axe de rotation (9) est l'axe du boulon transversal (8) qui est inséré latéralement dans la pièce de réception (5) et possède un tronçon (10) en forme de segment de cylindre qui présente le même rayon que la barre (2) à recevoir, et en ce que la surface (13) de la pièce formée supérieure (12) est cylindrique.

4. Dispositif d'ancrage (1) selon la revendication 1, caractérisé en ce que l'appui est configuré comme pièce formée inférieure (16).

5. Dispositif d'ancrage (1) selon la revendication 4, caractérisé en ce que l'axe de rotation (9) est situé à la hauteur de l'axe longitudinal (11) de la barre (2), la pièce formée inférieure (16) possédant les mêmes dimensions que la pièce formée supérieure (12), et la pièce formée inférieure et la pièce formée supérieure (16, 12) possèdent chacune une surface cylindrique (17) situées l'une en face de l'autre, par lesquelles elles peuvent être insérées en correspondance de forme dans la pièce de réception (5).

6. Dispositif d'ancrage (1) selon la revendication 5, caractérisé en ce que la pièce formée inférieure et la pièce formée supérieure (16, 12) sont fabriquées ensemble par tournage, et sont ensuite découpées ou fendues.

7. Dispositif d'ancrage (1) selon la revendication 5 caractérisé en ce que les surfaces (13, 17) de la pièce formée 16 et/ou de la pièce formée supérieure 12 sont des tronçons de sphère dont le centre est le point de rencontre de l'axe (11) de la barre et de l'axe de rotation (9).

8. Dispositif d'ancrage (1) selon l'une des revendications 1 à 7, caractérisé en ce que le bord inférieur de l'écrou (14) est constitué d'un matériau malléable et glissant qui est plus malléable que la surface (13) de la pièce formée supérieure (12).

9. Dispositif d'ancrage (1) selon la revendication 1, caractérisé en ce que l'écrou (14) possède sur le côté par lequel il est inséré par rotation dans les mâchoires (6, 6') un bord s'étendant obliquement vers l'extérieur.

10. Dispositif d'ancrage (1) selon l'une des revendications 1 à 9, caractérisé en ce que l'écrou (14) est constitué d'un matériau plus dur que la surface (13) de la pièce formée supérieure (12).

11. Dispositif d'ancrage (1) selon la revendication 10, caractérisé en ce que la surface (13) de la pièce formée supérieure (12) présente une structuration, de préférence sous la forme d'un moletage.

12. Dispositif d'ancrage (1) selon l'une des revendications 3 à 11, caractérisé en ce que le tronçon (10) de la pièce de réception (5) présente une surface lisse.

13. Dispositif d'ancrage (1) selon l'une des revendications 3 à 12, caractérisé en ce que le tronçon (10) de la pièce de réception (5) est doté d'un filet droit ou gauche par rapport à l'axe longitudinal (11) de la barre (2).

14. Dispositif pour la stabilisation de segments de la colonne vertébrale, comportant au moins deux dispositifs d'ancrage (1) selon l'une des revendications 1 à 13, caractérisé en ce que les dispositifs d'ancrage (1) sont reliés l'un à l'autre au moyen de barres (2).
